(19) Europäisches Patentamt
European Patent Office
Office européen des brevets

(11) **EP 0 565 076 B1**

(12) **EUROPEAN PATENT SPECIFICATION**

(45) Date of publication and mention
of the grant of the patent:
**03.07.1996 Bulletin 1996/27**

(51) Int Cl.$^6$: **C07C 68/00**, C07C 69/96

(21) Application number: **93105760.8**

(22) Date of filing: **07.04.1993**

(54) **Process for preparing carbonate**

Verfahren zur Herstellung eines Karbonates

Procédé pour la préparation d'un carbonate

(84) Designated Contracting States:
**DE FR GB**

(30) Priority: **07.04.1992 JP 129231/92**
**08.12.1992 JP 327990/92**

(43) Date of publication of application:
**13.10.1993 Bulletin 1993/41**

(73) Proprietor: **UBE INDUSTRIES, LTD.**
**Ube-shi, Yamaguchi-ken 755 (JP)**

(72) Inventors:
- **Matsuzaki, Tokuo, c/o Ube Research Laboratory**
  **Ube-shi, Yamaguchi-ken (JP)**
- **Shimamura, Tuneo,**
  **c/o Ube Research Laboratory**
  **Ube-shi, Yamaguchi-ken (JP)**
- **Toriyahara, Yosinobu,**
  **c/o Ube Research Laboratory**
  **Ube-shi, Yamaguchi-ken (JP)**
- **Yamasaki, Yoshinori,**
  **c/o Ube Research Laboratory**
  **Ube-shi, Yamaguchi-ken (JP)**

(74) Representative:
**Hansen, Bernd, Dr. Dipl.-Chem. et al**
**Hoffmann, Eitle & Partner**
**Patent- und Rechtsanwälte,**
**Postfach 81 04 20**
**81904 München (DE)**

(56) References cited:
**EP-A- 0 425 197        EP-A- 0 464 460**

## Description

This invention relates to a process for preparing a carbonic acid ester (carbonate) from carbon monoxide and a nitrous acid ester (nitrite) in which use of a catalyst can be industrially realized. Carbonate is a compound which is extremely useful as a starting material for organic syntheses of medicines and agricultural chemicals, and as an intermediate for syntheses of polycarbonates and urethanes.

As a method for preparing a diester of carbonic acid, it has been proposed a method of reacting carbon monoxide and a nitrite in the presence of a solid catalyst in which a platinum group metal or a compound thereof and other metal compound are carried on a carrier. The present inventors have previously proposed a method for preparing a diester of carbonic acid by contacting carbon monoxide and a nitrite in a vapor phase in the presence of the above solid catalyst as disclosed in Japanese Provisional Patent Publications No. 141243/1990 (which corresponds to U.S. Patent No. 5,162,563), No. 139152/1991, No. 290848/1991, No. 297443/1991, No. 297444/1991 and No. 297445/1991. However, there is a problem that activity of the catalyst is inevitably lowered with a long term use.

Also, a catalyst in which a platinum group metal is carried on is particularly widely used for various synthetic reactions other than the synthesis of carbonic diester and various regeneration methods of the catalyst have been proposed. In Japanese Provisional Patent Publication No. 240756/1991, carbonic diester is prepared from an alcohol, carbon monoxide and oxygen in the presence of a catalyst in which a platinum group compound is carried and a deactivated catalyst is regenerated by subjecting to heat treatment and weak acid treatment. However, said regenerating methods of a catalyst are of course different from each other with regard to respective catalysts used in different synthetic methods of carbonic diester. That is, the regenerating method of a catalyst disclosed in the above reference cannot be applied to a catalyst used for a different synthetic method of carbonic diester.

In the method of preparing carbonic diester from carbon monoxide and a nitrite, a solid catalyst in which a platinum group metal or a platinum group metal compound and other metal compound are carried on a carrier gradually lowers in activity with a long period of use and a yield of carbonic diester which is an objective compound is inevitably worsened. Thus, in order to use a catalyst for a long period of time, it is extremely important to use the catalyst repeatedly by regenerating the catalyst when catalyst activity thereof is lowered since a platinum group metal or a platinum group metal compound is extremely expensive so that loss of them should be avoided.

## SUMMARY OF THE INVENTION

An object of the present invention is to provide an industrially advantageous process for preparing a carbonate in which a deactivated catalyst can be regenerated by a simple and easy method, the catalyst of which is used in a process for preparing a carbonic acid diester by reacting carbon monoxide and a nitrite in the presence of a solid catalyst in which a platinum group metal or a platinum group metal compound and optionally other metal compound are carried on a carrier. In a practical solid bed vapor phase process, it is particularly important that a catalyst can be used for a long period of time discontinuously and can be regenerated with a state filled in a reactor whereby continuous operation is realized.

The present inventors have studied intensively, in a process for preparing a carbonic acid diester by reacting carbon monoxide and a nitrite in the presence of a solid catalyst in which at least one of a platinum group metal and a platinum group metal compound, and optionally other metal compound, are carried on a carrier, in order to solve the problems involved in the conventional preparation methods. As a result, they have found that deactivated catalyst components can be regenerated by treating with a hydrogen and hydrogen chloride simultaneously or successively so that the catalyst can be used repeatedly by regenerating it as mentioned above when it is deactivated whereby an industrially available process can be provided.

That is, the present invention is a process for preparing diester of carbonic acid from carbon monoxide and a nitrite in the presence of a solid catalyst in which at least one of a platinum group metal and a platinum group metal compound, and optionally other metal compound, is carried on a carrier, characterized in that a deactivated catalyst is regenerated by treating with hydrogen and with hydrogen chloride simultaneously or successively whereby the catalyst can be used repeatedly.

In the process of the present invention, it may be also possible that a deactivated catalyst is treated with hydrogen, and also successively or simultaneously treated with a mixture of hydrogen chloride and a nitrite whereby the regenerated catalyst can be used for the above reaction repeatedly.

## DESCRIPTION OF THE PREFERRED EMBODIMENTS

In the following, the present invention is to be described in more detail.

Of the solid catalysts to be used in the present invention, the platinum group metals may include, for example, platinum, palladium, ruthenium and rhodium. The platinum group metal compound may include, for example, a halide (i.e. fluoride, chloride, bromide and iodide) or a sulfate of platinum, palladium, ruthenium and rhodium, and palladium chloride is particularly preferred. The amount of these component to be carried on a carrier is in terms of the metal 0.1 to 10 % by weight, preferably 0.5 to 2 % by weight based on the weight of the carrier.

In the process of the present invention, in addition to the platinum group metal and/or the platinum group metal compound, other metal compound may be optionally used in combination.

As the other metal compound optionally contained, there may be mentioned, for example, a compound of a metal such as iron, copper, bismuth, cobalt, nickel, tin, vanadium, molybdenum, tungsten, an alkali metal and an alkaline earth metal.

The other metal compound may include, for example, a halide (i.e. fluoride, chloride, bromide and iodide), nitrate, sulfate, phosphate or acetate of a metal such as iron, copper, bismuth, cobalt, nickel and tin, preferably a halide of these metals, particularly preferably a chloride of these metals. The amount of these metals to be carried on a carrier is in terms of the metal 0.1 to 50 gram atomic equivalents, preferably 1 to 10 gram atomic equivalents.

The other metal compound may further include an oxide, metal acid, metal acid salt or metal acid ammonium of a metal such as vanadium, molybdenum and tungsten, and a metal acid ammonium such as ammonium vanadate, ammonium molybdate and ammonium tungstate is preferably used. The amount of these metals to be carried on a carrier is in terms of the metal and based on the platinum group metal 0.1 to 20 gram atomic equivalents, preferably 0.5 to 5 gram atomic equivalents.

Further, as the other metal compounds, there may be mentioned an alkali metal salt or an alkaline earth metal salt of an organic acid, a halide, particularly a fluoride or a chloride of an alkali metal, and an alkali metal salt or an alkaline earth metal salt of a phosphoric acid.

As an alkali metal salt or an alkaline earth metal salt of an organic acid, there may be mentioned a formate or an acetate thereof, and potassium acetate is particularly suitable used. The amount of the formate or the acetate to be carried on a carrier is in terms of the metal and based on the platinum group metal 0.1 to 20 gram atomic equivalents, particularly preferably 0.5 to 10 gram atomic equivalents.

As a fluoride or a chloride of an alkali metal, potassium fluoride and potassium chloride may be used. The amount of these compounds to be carried on a carrier is in terms of metal and based on the platinum group metal 0.1 to 20 gram atomic equivalents, particularly preferably 0.5 to 10 gram atomic equivalents.

As an alkali metal salt or an alkaline earth metal salt of phosphoric acid, potassium hydrogen phosphate and strontium hydrogen phosphate are suitable used. The amount of these compounds to be carried on a carrier is in terms of the metal and based on the platinum group metal 0.1 to 20 gram atomic equivalents, particularly preferably 0.5 to 10 gram atomic equivalents.

As the carrier on which the above platinum group metal and/or platinum group metal compound, and optionally the other metal compound, are carried, diatomaceous earth, activated charcoal, silicon carbide, titania, alumina (e.g. $\alpha$-, $\beta$- and $\gamma$-alumina) and silica alumina are suitably used, but activated charcoal is preferred. The method of carrying the above respective catalyst component on the carrier is not particularly limited and usually employed methods such as impregnation method (dip-adsorption method), kneading method, precipitation method, evaporation to dryness method and coprecipitation method may be used, but in the present invention, the catalyst is preferably prepared by the impregnation method (dip-adsorption method) and evaporation to dryness method since they are simple and easy. The above catalyst components may be carried on a carrier simultaneously or successively.

In the present invention, the above catalyst is used in the form of powder, granules or a molded product and the size thereof is not particularly limited. In the case of powder, those having 20 to 100 $\mu$m in diameter are generally and suitably used and in the case of granules, those having 4 to 200 mesh or so are generally preferably used. In the case of the molded product, those having about 4 to 6 mm in diameter are generally preferred.

As a nitrite to be used in the present invention, there may be suitably mentioned a nitrite of a lower aliphatic monohydric alcohol having 1 to 4 carbon atoms such as methyl nitrite, ethyl nitrite, n- (or iso)propyl nitrite and n-(or iso or sec-)butyl nitrite; a nitrite of an alicyclic alcohol such as cyclohexyl nitrite; a nitrite of an aralkyl alcohol such as benzyl nitrite and phenylethyl nitrite, but particularly preferably a nitrite of a lower aliphatic monohydric alcohol having 1 to 4 carbon atoms, and above all, methyl nitrite and ethyl nitrite are most preferred. Also, carbon monoxide may be used as a starting material not only a high purity carbon monoxide gas but also those diluted with a gas inert to the synthetic reaction such as nitrogen, argon and carbon dioxide.

In the process of the present invention, the contact reaction of carbon monoxide and a nitrite can be carried out, for example, at a temperature of 0 to 200 °C, preferably 50 to 140 °C under normal pressure or under pressure without any problem. Preferably, it can be carried out at a pressure of 1 to 20 $kg/cm^2G$ and a temperature range of 50 to 140 °C. As a reaction system, the reaction may be carried out in a vapor phase by either a batch system or a continuous system, but the continuous system is industrially advantageous. Also, as an existing form of the catalyst in the reaction system, the reaction may be practiced by using a reactor either of a fixed bed or a fluidized bed. A space velocity of a gas containing carbon monoxide and a nitrite to be fed in the above reactor is preferably in the range of 500 to 20000 $hr^{-1}$, more preferably in the range of 2000 to 15000 $hr^{-1}$.

EP 0 565 076 B1

As a regeneration treatment method of the solid catalyst according to the present invention, there may be mentioned, for example, (i) a method of regenerating a deactivated solid catalyst comprising the steps of treating with a gas containing hydrogen (hereinafter also referred to as "a hydrogen gas") and then treating with a gas containing hydrogen chloride (hereinafter also referred to as "a hydrogen chloride gas"), (ii) a method of regenerating the same comprising the steps of treating with a hydrogen gas, treating with a hydrogen chloride gas, and then treating with a gas containing a nitric acid ester (hereinafter also referred to as "a nitrate gas"), (iii) a method of regenerating the same comprising the steps of treating with a hydrogen gas, and then treating with a mixed gas containing hydrogen chloride and a nitrite, and (iv) a method of regenerating the same comprising the step of treating with a mixed gas containing hydrogen and hydrogen chloride.

The catalyst regenerating treatment method of the present invention comprises the steps of treating the deactivated catalyst with a hydrogen gas and treating the same with a hydrogen chloride gas. Regeneration using a hydrogen gas can be carried out by treating the deactivated catalyst in a hydrogen gas stream. A hydrogen gas to be fed is not necessarily pure one but may be a hydrogen gas diluted with an inert gas such as nitrogen and argon. The concentration of hydrogen to be used is in the range of 5 to 100 %, particularly preferably 20 to 100 %.

Treatment with a hydrogen gas is carried out under the conditions of a space velocity of a gas containing hydrogen desirably in the range of 50 to 20000 hr$^{-1}$, more preferably in the range of 100 to 10000 hr$^{-1}$. The pressure to effect the hydrogen treatment is preferably 1 to 100 atm, particularly preferably 1 to 20 atm. When heating is carried out at too low temperature or too high temperature, effects cannot sufficiently be obtained so that the treatment is preferably carried out at a temperature of 70 to 500 °C, more preferably 100 to 450 °C, for a reaction time of several hours to several ten hours.

The catalyst treated with hydrogen is then applied to the treatment with hydrogen chloride. The hydrogen chloride gas to be used includes not only a pure gas but also a hydrogen chloride gas diluted with an inert gas such as nitrogen and argon. The concentration of the hydrogen chloride is preferably 20 ppm to 100 %, particularly preferably 100 to 50000 ppm. A space velocity of a gas containing hydrogen chloride is desirably in the range of 40 to 20000 hr$^{-1}$, particularly preferably in the range of 100 to 10000 hr$^{-1}$.

The pressure to effect the treatment with hydrogen chloride is suitably 1 to 20 atm, particularly preferably 1 to 5 atm. The treatment temperature with hydrogen chloride is suitable 15 to 500 °C, particularly preferably 50 to 400 °C, and the treatment time is desirably 0.01 to 100 hours, particularly preferably 0.1 to 50 hours. Also, the above treatment with hydrogen and the treatment with hydrogen chloride may be carried out simultaneously, and in this case, a deactivated catalyst is treated with a mixed gas of a hydrogen gas and a hydrogen chloride gas under the conditions as mentioned above so that the object can be sufficiently accomplished.

In the treatment with a mixed gas containing hydrogen chloride and a nitrite or with a nitrite gas, the concentration of the nitrite is preferably 0.5 to 20 %, more preferably 2 to 15 %. A space velocity of the mixed gas or the nitrite gas is preferably in the range of 10 to 20000 hr$^{-1}$, more preferably 40 to 10000 hr$^{-1}$.

When the treatment is carried out with the mixed gas of hydrogen chloride and a nitrite or with a nitrite gas, the pressure is preferably 1 to 20 atm, more preferably 1 to 5 atm. Also, the temperature of the treatment is preferably carried out at 15 to 300 °C, more preferably 25 to 250 °C, and the treatment time is preferably 0.01 to 100 hours, more preferably 0.1 to 50 hours.

In regenerating the solid catalyst according to the present invention, in order to treat a deactivated solid catalyst with hydrogen and hydrogen chloride, it is preferred that the deactivated solid catalyst is purged with a suitable inert gas such as a nitrogen gas and an argon gas for a suitable time. Also, it is preferred that a filling bed of the deactivated solid catalyst is cooled to at around the room temperature before effecting the treatment.

EXAMPLES

In the following, the process of the present invention is explained specifically by referring to Examples, but they are one of the embodiments of the present invention and the present invention is not limited by these Examples.

A space time yield (STY) Y (g/ℓ·hr) in each Example or Comparative example is calculated from the following equation:

$$Y = a/(b \times \theta) \qquad (a)$$

wherein θ (hr) is a contact reaction time of carbon monoxide and a nitrite, $\underline{a}$ (g) is an amount of a diester of carbonic acid and $\underline{b}$ (ℓ) is a filled amount of the catalyst to the reactor.

Also, an activity decreasing coefficient Da (hr$^{-1}$) in each Example or Comparative example is calculated from the following equation (c) after calculating k according to the following equation (b) from, under predetermined reaction conditions, a space time yield $Y_0$ (g/ℓ·hr) at an initial stage of the reaction (after 2 hours from initiation of the reaction) and a space time yield $Y_t$ (g/ℓ·hr) at a time t hours lapsed from initiation of the reaction.

4

$$Y_t = Y_0 \cdot \exp(-kt) \tag{b}$$

$$Da = 100 \times k \tag{c}$$

## Example 1

(Preparation of a catalyst)

In 100 ml of 5N hydrochloric acid was dissolved 0.33 g of palladium chloride, and after dipping 20 g of granular activated charcoal therein, the activated charcoal was filtered, washed with water and dried at 200 °C to produce a catalyst ($PdCl_2$/C) in which palladium chloride alone had been carried on the activated charcoal.

(Synthesis of dimethyl carbonate)

In a vapor phase reactor (attached with an outer jacket) having an inner diameter of 20 mm was filled 2.5 ml of the above catalyst, and then the reactor was fixed vertically and a heating medium was circulated in the reactor jacket whereby the temperature in a catalyst layer was controlled to 120 °C. From the upper portion of the reactor, a mixed gas of a gas containing methyl nitrite synthesized from nitrogen monoxide, oxygen and methanol, and carbon monoxide, i.e. a mixed gas comprising the composition of 8 % by volume of methyl nitrite, 8 % by volume of carbon monoxide, 3 % by volume of nitrogen monoxide, 10 % by volume of methanol and 71 % by volume of nitrogen was fed with a space velocity (GHSV) of 8000 $hr^{-1}$ under normal pressure to effect the reaction.

Then, the reaction product passed through the reactor was captured by passing through ice-cold methanol. The resulting captured solution was analyzed by gas chromatography to obtain the STY of dimethyl carbonate after 2 hours from initiation of the reaction of 230 g/$\ell$·hr. The reaction temperature was gradually increased so as to maintain the STY. The reaction was stopped at 110 hours from initiation of the reaction since activity of the catalyst could not be maintained even when raising the reaction temperature.

(Regeneration of the catalyst)

After termination of the synthesis of dimethyl carbonate, the reactor was cooled to room temperature by passing $N_2$ gas therethrough, and then $N_2$ gas was stopped. Then, $H_2$ gas was fed to the reactor with a flow rate of 20 ml/min and the reactor was elevated to 400 °C over 2 hours, and then maintained at 400 °C for 2 hours. Thereafter, the reactor was cooled to room temperature by passing $\underline{H}_2$ gas therethrough. Subsequently, the reactor was elevated to 120 °C over 2 hours while feeding HCl gas (concentration: 1000 ppm) diluted with $N_2$ gas with a flow rate of 180 ml/min and maintained at the temperature of 120 °C for 8 hours. After the treatment, $N_2$ gas was passed through the reactor to cool it to the room temperature. By using the regenerated catalyst, dimethyl carbonate was synthesized in accordance with the above first reaction. As the results, the STY (2 hour) was 226 g/$\ell$·hr, and it could be confirmed that the regenerated catalyst had the same activity as that of the newly prepared catalyst. The results are shown in Table 1.

## Example 2

(Preparation of a catalyst)

In 100 ml of 5N hydrochloric acid were dissolved 0.33 g of palladium chloride and 0.64 g of cupric chloride dihydride, and after dipping 20 g of granular activated charcoal therein, the activated charcoal was filtered, washed with water and dried at 200 °C to produce a catalyst ($PdCl_2$-$CuCl_2$/C) in which palladium chloride and cupric chloride had been carried on the activated charcoal.

(Synthesis of dimethyl carbonate)

Synthesis of dimethyl carbonate was carried out in the same manner as in Example 1 except for changing the catalyst used in Example 1 to the catalyst obtained above. The STY of dimethyl carbonate after 2 hours from initiation of the reaction was 380 g/$\ell$·hr. The reaction temperature was gradually increased so as to maintain the STY. The reaction was stopped at 90 hours from initiation of the reaction since activity of the catalyst could not be maintained even when raising the reaction temperature.

(Regeneration of the catalyst)

In the same manner as in Example 1 except for changing the catalyst, regeneration of the catalyst was carried out. By using the regenerated catalyst, dimethyl carbonate was synthesized in accordance with the above first reaction. As the results, the STY (2 hour) was 377 g/$\ell$·hr, and it could be confirmed that the regenerated catalyst had the same activity as that of the newly prepared catalyst.

Example 3

(Preparation of a catalyst)

In 70 ml of a 28 % by weight aqueous ammonia were dissolved 0.33 g of palladium chloride, 0.64 g of cupric chloride (CuCl$_2$·2H$_2$O) and 0.80 g of ammonium molybdate ((NH$_4$)$_6$Mo$_7$O$_{24}$· 4H$_2$O) under heating at 80 to 90 °C to prepare a "solution containing Pd, Cu and Mo". On the other hand, 20 g of granular activated charcoal (C) with a size of 0.5 to 0.7 mm was dipped in a 28 % by weight aqueous ammonia, and then the above "solution containing Pd, Cu and Mo" was added to the mixture and allowed to stand for one hour. Subsequently, water component in the activated charcoal was removed by evaporation at 80 °C under reduced pressure, and the activated charcoal was dried at 200 °C under nitrogen atmosphere to produce a catalyst. The composition of the resulting catalyst was PdCl$_2$-CuCl$_2$-(NH$_4$)$_6$Mo$_7$O$_{24}$/C and the carried amount of the metal compounds in the catalyst were 1 % by weight of Pd in terms of a metal based on the carrier and Pd : Cu : Mo = 1 : 2 : 2.1 (atomic ratio).

(Synthesis of dimethyl carbonate)

Synthesis of dimethyl carbonate was carried out in the same manner as in Example 1 except for changing the catalyst used in Example 1 to the catalyst obtained above. The STY of dimethyl carbonate after 2 hours from initiation of the reaction was 465 g/$\ell$·hr. The reaction temperature was gradually increased so as to maintain the STY. The reaction was stopped at 300 hours from initiation of the reaction since activity of the catalyst could not be maintained even when raising the reaction temperature.

(Regeneration of the catalyst)

In the same manner as in Example 1 except for changing the catalyst, regeneration of the catalyst was carried out. By using the regenerated catalyst, dimethyl carbonate was synthesized in accordance with the above first reaction. As the results, the STY (2 hour) was 457 g/$\ell$·hr, and it could be confirmed that the regenerated catalyst had the same activity as that of the newly prepared catalyst.

Example 4

(Preparation of a catalyst)

In 70 ml of a 25 % by weight aqueous ammonia were dissolved 0.33 g of palladium chloride, 0.64 g of cupric chloride (CuCl$_2$·2H$_2$O), 0.80 g of ammonium molybdate ((NH$_4$)$_6$Mo$_7$O$_{24}$· 4H$_2$O) and 0.227 g of potassium chloride under heating at 80 to 90 °C to prepare a "solution containing Pd, Cu and Mo". On the other hand, 20 g of granular activated charcoal (C) with a size of 0.5 to 0.7 mm was dipped in a 25 % by weight aqueous ammonia, and then the above "solution containing Pd, Cu and Mo" was added to the mixture and allowed to stand for one hour. Subsequently, water component in the activated charcoal was removed by evaporation at 80 °C under reduced pressure, and the activated charcoal was dried at 200 °C under nitrogen atmosphere to produce a catalyst. The composition of the resulting catalyst was PdCl$_2$-CuCl$_2$-(NH$_4$)$_6$Mo$_7$O$_{24}$-KCl/C and the carried amount of the metal compounds in the catalyst were 1 % by weight of Pd in terms of a metal based on the carrier and Pd : Cu : Mo : KCl = 1 : 2 : 2.1 : 2 (atomic ratio).

(Synthesis of dimethyl carbonate)

Synthesis of dimethyl carbonate was carried out in the same manner as in Example 1 except for changing the catalyst used in Example 1 to the catalyst obtained above. The STY of dimethyl carbonate after 2 hours from initiation of the reaction was 410 g/$\ell$·hr. The reaction temperature was gradually increased so as to maintain the STY. The reaction was stopped at 500 hours from initiation of the reaction since activity of the catalyst could not be maintained even when raising the reaction temperature.

(Regeneration of the catalyst)

In the same manner as in Example 1 except for changing the catalyst, regeneration of the catalyst was carried out. By using the regenerated catalyst, dimethyl carbonate was synthesized in accordance with the above first reaction. As the results, the STY (2 hour) was 405 g/ℓ·hr, and it could be confirmed that the regenerated catalyst had the same activity as that of the newly prepared catalyst.

Examples 5, 6 and 9

(Preparation of catalysts)

In the same manner as in Example 3, catalysts were produced.

(Synthesis of dimethyl carbonate)

In the same manner as in Example 3, syntheses of dimethyl carbonate were carried out.

(Regeneration of the catalysts)

In the same manner as in Example 1 except for changing the treatment temperature with hydrogen at generation of the catalyst, the catalysts were regenerated. It could be confirmed that the regenerated catalysts had the same activity as those of the newly prepared catalysts.

Examples 7 and 8

(Preparation of catalysts)

In the same manner as in Example 3, catalysts were produced.

(Synthesis of dimethyl carbonate)

In the same manner as in Example 3, syntheses of dimethyl carbonate were carried out.

(Regeneration of the catalysts)

In the same manner as in Example 1 except for changing the treatment temperature with hydrogen chloride at generation of the catalyst to 60 °C or 250 °C, the catalysts were regenerated. It could be confirmed that the regenerated catalysts had the same activity as those of the newly prepared catalysts.

Example 10

(Preparation of catalysts)

In the same manner as in Example 3, a catalyst was produced.

(Synthesis of dimethyl carbonate)

In the same manner as in Example 3, synthesis of dimethyl carbonate was carried out.

(Regeneration of the catalysts)

After termination of the synthesis of dimethyl carbonate, the reactor was cooled to room temperature by passing $N_2$ gas therethrough. Subsequently, a mixed gas of $N_2$ and $H_2$ containing 1000 ppm of HCl ($N_2$ : $H_2$ = 1 : 9 volume ratio) was fed to the reactor with a flow rate of 50 ml/min and the reactor was elevated to 150 °C over one hour, and then maintained at 150 °C for 16 hours. Thereafter, the mixed gas was stopped and the reactor was cooled to room temperature by passing $N_2$ gas therethrough over one hour. It could be confirmed that the regenerated catalyst had the same activity as that of the newly prepared catalyst.

## Example 11

In the same manner as in Example 1 except for changing HCl gas in the regeneration of the catalyst with a mixed gas of HCl gas and a nitrite as shown below, preparation of a catalyst, synthesis of dimethyl carbonate and regeneration of the catalyst were carried out. That is, in the regeneration of the catalyst, after treatment with $H_2$ gas, the reactor was elevated to 120 °C over 2 hours while feeding a mixed gas of HCl gas (concentration: 1000 ppm) and methyl nitrite (concentration: 10 %) diluted with $N_2$ gas with a flow rate of 180 ml/min and maintained at the temperature of 120 °C for 8 hours. After the treatment, $N_2$ gas was passed through the reactor to cool it to the room temperature.

By using the regenerated solid catalyst (regenerated catalyst), dimethyl carbonate was synthesized in accordance with the above first reaction. As the results, the STY (2 hour) was 235 g/ℓ·hr, and STY after 10 hours from initiation of the reaction was 115 g/ℓ·hr. As the results, it could be found that an activity decreasing coefficient Da in the synthetic reaction using the above regenerated catalyst was 6.4 $hr^{-1}$. The results are shown in Table 1.

## Example 12

In the same manner as in Example 2, preparation of the catalyst and synthesis of dimethyl carbonate were carried out. Then, in the same manner as in Example 11 except for changing the solid catalyst used in Example 11 with a deactivated solid catalyst ($PdCl_2$-$CuCl_2$/C), regeneration of the catalyst was carried out.

By using the regenerated catalyst, dimethyl carbonate was synthesized in accordance with the above first reaction. As the results, the STY (2 hour) was 373 g/ℓ·hr, and STY after 10 hours from initiation of the reaction was 223 g/ℓ·hr. As the results, it could be found that an activity decreasing coefficient Da in the synthetic reaction using the above regenerated catalyst was 5.1 $hr^{-1}$. The results are shown in Table 1.

## Example 13

In the same manner as in Example 3, preparation of the catalyst and synthesis of dimethyl carbonate were carried out. Then, in the same manner as in Example 11 except for changing the solid catalyst used in Example 11 with a deactivated solid catalyst ($PdCl_2$-$CuCl_2$-$(NH_4)_6Mo_7O_{24}$/C), regeneration of the catalyst was carried out.

By using the regenerated catalyst, dimethyl carbonate was synthesized in accordance with the above first reaction. As the results, the STY (2 hour) was 467 g/ℓ·hr, and STY after 10 hours from initiation of the reaction was 422 g/ℓ·hr. As the results, it could be found that an activity decreasing coefficient Da in the synthetic reaction using the above regenerated catalyst was 1.2 $hr^{-1}$. The results are shown in Table 1.

## Example 14

In the same manner as in Example 4, preparation of the catalyst and synthesis of dimethyl carbonate were carried out. Then, in the same manner as in Example 11 except for changing the solid catalyst used in Example 11 with a deactivated solid catalyst ($PdCl_2$-$CuCl_2$-$(NH_4)_6Mo_7O_{24}$-KCl/C), regeneration of the catalyst was carried out.

By using the regenerated catalyst, dimethyl carbonate was synthesized in accordance with the above first reaction. As the results, the STY (2 hour) was 413 g/ℓ·hr, and STY after 10 hours from initiation of the reaction was 390 g/ℓ·hr. As the results, it could be found that an activity decreasing coefficient Da in the synthetic reaction using the above regenerated catalyst was 0.7 $hr^{-1}$. The results are shown in Table 1.

## Examples 15 and 16

In the same manner as in Example 3, preparation of the catalyst and synthesis of dimethyl carbonate were carried out. Then, in the same manner as in Example 13 except for changing the treatment temperature with a mixed gas of a hydrogen chloride gas and methyl nitrite to 60 °C (Example 15) or 25 °C (Example 16), regeneration of the catalyst was carried out.

By using the regenerated catalyst, dimethyl carbonate was synthesized in accordance with the above first reaction. As the results, it could be confirmed that the regenerated catalysts had the same activity with a newly prepared catalyst and activity decreasing ratio was little. The results are shown in Table 1.

## Examples 17 and 18

In the same manner as in Example 3, preparation of the catalyst and synthesis of dimethyl carbonate were carried out. Then, in the same manner as in Example 13 except for changing the treatment temperature with a HCl gas to 60 °C (Example 17) or 250 °C (Example 18), regeneration of the catalyst was carried out.

By using the regenerated catalyst, dimethyl carbonate was synthesized in accordance with the above first reaction. The results are shown in Table 1.

Comparative example 1

In the same manner as in Example 3 except for effecting hydrogen treatment for the catalyst regeneration, preparation of a catalyst, synthesis of dimethyl carbonate and regeneration of the catalyst were carried out. The results are shown in Table 1.

Comparative example 2

In the same manner as in Example 3 except for effecting hydrogen chloride treatment for the catalyst regeneration, preparation of a catalyst, synthesis of dimethyl carbonate and regeneration of the catalyst were carried out.

Comparative example 3

In the same manner as in Example 1 except for effecting regeneration of the catalyst, preparation of a catalyst and synthesis of dimethyl carbonate were carried out to obtain a deactivated $PdCl_2$/C catalyst.

(Synthesis of dimethyl carbonate)

In a vapor phase reactor (attached with an outer jacket) having an inner diameter of 20 mm was filled 2.5 ml of the above catalyst, and then the reactor was fixed vertically and a heating medium was circulated in the reactor jacket whereby the temperature in a catalyst layer was controlled to 120 °C. From the upper portion of the reactor, a mixed gas of a gas containing methyl nitrite synthesized from nitrogen monoxide, oxygen and methanol, and carbon monoxide, i.e. a mixed gas comprising the composition of 8 % by volume of methyl nitrite, 8 % by volume of carbon monoxide, 3 % by volume of nitrogen monoxide, 10 % by volume of methanol and 71 % by volume of nitrogen was fed with a space velocity (GHSV) of 8000 hr$^{-1}$ under normal pressure to effect the reaction.

Then, the reaction product passed through the reactor was captured by passing through ice-cold methanol. The resulting captured solution was analyzed by gas chromatography to obtain the STY of dimethyl carbonate after 2 hours from initiation of the reaction of 6 g/$\ell$·hr.

Comparative example 4

In the same manner as in Example 2 except for effecting regeneration of the catalyst, preparation of a catalyst and synthesis of dimethyl carbonate were carried out to obtain a deactivated $PdCl_2$-$CuCl_2$/C catalyst.

(Synthesis of dimethyl carbonate)

In the same manner as in Comparative example 3, synthesis of dimethyl carbonate and analysis thereof were carried out. As the results, the STY of dimethyl carbonate after 2 hours from initiation of the reaction was 5 g/$\ell$·hr.

Comparative example 5

In the same manner as in Example 3 except for effecting regeneration of the catalyst, preparation of a catalyst and synthesis of dimethyl carbonate were carried out to obtain a deactivated $PdCl_2$-$CuCl_2$-$(NH_4)_6Mo_7O_{24}$/C catalyst.

(Synthesis of dimethyl carbonate)

In the same manner as in Comparative example 3, synthesis of dimethyl carbonate and analysis thereof were carried out. As the results, the STY of dimethyl carbonate after 2 hours from initiation of the reaction was 10 g/$\ell$·hr.

Comparative example 6

In the same manner as in Example 4 except for effecting regeneration of the catalyst, preparation of a catalyst and synthesis of dimethyl carbonate were carried out to obtain a deactivated $PdCl_2$-$CuCl_2$-$(NH_4)_6Mo_7O_{24}$-$KCl$/C catalyst.

(Synthesis of dimethyl carbonate)

In the same manner as in Comparative example 3, synthesis of dimethyl carbonate and analysis thereof were carried out. As the results, the STY of dimethyl carbonate after 2 hours from initiation of the reaction was 7 g/ℓ·hr.

According to the process of the present invention, a deactivated catalyst used for preparing diester of carbonic acid from carbon monoxide and a nitrite in the presence of a solid catalyst in which at least one of a platinum group metal, a platinum group metal compound and other metal compound is carried on a carrier can be effectively regenerated, and thus, the catalyst can be used for a long period of time and lifetime of the catalyst can be markedly elongated.

EP 0 565 076 B1

Table 1

| No. | Catalyst | Regeneration of catalyst | | DMC formed rate Y (g/l·hr) | Activity decreased coefficient Da ($hr^{-1}$) |
|---|---|---|---|---|---|
| | | $H_2$ treatment | HCl treatment | | |
| Example 1 | $PdCl_2$/C | Normal temp. $\xrightarrow[\text{2 hr}]{H_2}$ 400 °C $\xrightarrow[\text{2 hr}]{H_2}$ 400 °C $\xrightarrow[\text{2 hr}]{H_2}$ Normal temp. | Normal temp. $\xrightarrow[\text{0.5hr}]{HCl}$ 120 °C $\xrightarrow[\text{8 hr}]{HCl}$ 120 °C $\xrightarrow[\text{0.5hr}]{N_2}$ Normal temp. | 226 | 8.3 |
| Example 2 | $PdCl_2$-$CuCl_2$/C | Same as above | Same as above | 377 | 8.2 |
| Example 3 | $PdCl_2$-$CuCl_2$-$(NH_4)_6Mo_7O_{24}$/C | Same as above | Same as above | 457 | 2.5 |
| Example 4 | $PdCl_2$-$CuCl_2$-$(NH_4)_6Mo_7O_{24}$-KCl/C | Same as above | Same as above | 405 | 1.2 |
| Example 5 | $PdCl_2$-$CuCl_2$-$(NH_4)_6Mo_7O_{24}$/C | Normal temp. $\xrightarrow[\text{2 hr}]{H_2}$ 300 °C $\xrightarrow[\text{2 hr}]{H_2}$ 300 °C $\xrightarrow[\text{2 hr}]{H_2}$ Normal temp. | Same as above | 474 | 2.6 |
| Example 6 | $PdCl_2$-$CuCl_2$-$(NH_4)_6Mo_7O_{24}$/C | Normal temp. $\xrightarrow[\text{1 hr}]{H_2}$ 200 °C $\xrightarrow[\text{2 hr}]{H_2}$ 200 °C $\xrightarrow[\text{1 hr}]{H_2}$ Normal temp. | Same as above | 467 | 2.2 |
| Example 7 | $PdCl_2$-$CuCl_2$-$(NH_4)_6Mo_7O_{24}$/C | Same as above | Normal temp. $\xrightarrow[\text{0.5hr}]{HCl}$ 60 °C $\xrightarrow[\text{8 hr}]{HCl}$ 60 °C $\xrightarrow[\text{0.5hr}]{N_2}$ Normal temp. | 450 | 2.8 |

11

EP 0 565 076 B1

Table 1 (contd)

| No. | Catalyst | Regeneration of catalyst | | DMC formed rate Y (g/l·hr) | Activity decreased coefficient Da (hr$^{-1}$) |
|---|---|---|---|---|---|
| | | $H_2$ treatment | HCl treatment | | |
| Example 8 | PdCl$_2$-CuCl$_2$-(NH$_4$)$_6$Mo$_7$O$_{24}$/C | Normal temp. $\xrightarrow[\text{1 hr}]{H_2}$ 200 °C $\xrightarrow[\text{2 hr}]{H_2}$  200 °C $\xrightarrow[\text{1 hr}]{H_2}$ Normal temp. | Normal temp. $\xrightarrow[\text{2 hr}]{HCl}$ 250 °C $\xrightarrow[\text{2 hr}]{HCl}$  250 °C $\xrightarrow[\text{2 hr}]{N_2}$ Normal temp. | 467 | 2.4 |
| Example 9 | PdCl$_2$-CuCl$_2$-(NH$_4$)$_6$Mo$_7$O$_{24}$/C | Normal temp. $\xrightarrow[\text{2 hr}]{H_2}$ 150 °C $\xrightarrow[\text{2 hr}]{H_2}$  150 °C $\xrightarrow[\text{2 hr}]{H_2}$ Normal temp. | Normal temp. $\xrightarrow[\text{0.5hr}]{HCl}$ 120 °C $\xrightarrow[\text{8 hr}]{HCl}$  120 °C $\xrightarrow[\text{0.5hr}]{N_2}$ Normal temp. | 408 | 2.5 |
| Example 10 | PdCl$_2$-CuCl$_2$-(NH$_4$)$_6$Mo$_7$O$_{24}$/C | Normal temp. $\xrightarrow[\text{2 hr}]{HCl/H_2/N_2}$ 150 °C $\xrightarrow[\text{2 hr}]{HCl/H_2/N_2}$  150 °C $\xrightarrow[\text{1 hr}]{N_2}$ Normal temp. | (Simultaneous treatment) | 460 | — |
| Example 11 | PdCl$_2$/C | Normal temp. $\xrightarrow[\text{2 hr}]{H_2}$ 400 °C $\xrightarrow[\text{2 hr}]{H_2}$  400 °C $\xrightarrow[\text{2 hr}]{H_2}$ Normal temp. | Normal temp. $\xrightarrow[\text{0.5hr}]{HCl-CH_3ONO}$ 120 °C  HCl-CH$_3$ONO $\xrightarrow[\text{8 hr}]{}$ 120°C $\xrightarrow[\text{0.5hr}]{N_2}$ Normal temp. | 235 | 6.4 |
| Example 12 | PdCl$_2$-CuCl$_2$/C | Same as above | Same as above | 373 | 5.1 |
| Example 13 | PdCl$_2$-CuCl$_2$-(NH$_4$)$_6$Mo$_7$O$_{24}$/C | Same as above | Same as above | 467 | 1.2 |
| Example 14 | PdCl$_2$-CuCl$_2$-(NH$_4$)$_6$Mo$_7$O$_{24}$-KCl/C | Same as above | Same as above | 413 | 0.7 |

Table 1 (contd)

| No. | Catalyst | Regeneration of catalyst | | DMC formed rate Y (g/l·hr) | Activity decreased coefficient Da (hr$^{-1}$) |
|---|---|---|---|---|---|
| | | H$_2$ treatment | HCl treatment | | |
| Example 15 | PdCl$_2$-CuCl$_2$-(NH$_4$)$_6$Mo$_7$O$_{24}$/C | Normal temp. $\xrightarrow[\text{2 hr}]{\text{H}_2}$ 400 ·C $\xrightarrow[\text{2 hr}]{\text{H}_2}$ 400 ·C $\xrightarrow[\text{2 hr}]{\text{H}_2}$ Normal temp. | Normal temp. $\xrightarrow[\text{15 min}]{\text{HCl-CH}_3\text{ONO}}$ 60 ·C $\xrightarrow[\text{8 hr}]{\text{HCl-CH}_3\text{ONO}}$ 60·C $\xrightarrow[\text{15min}]{\text{N}_2}$ Normal temp. | 445 | 1.1 |
| Example 16 | PdCl$_2$-CuCl$_2$-(NH$_4$)$_6$Mo$_7$O$_{24}$/C | Same as above | Normal temp. $\xrightarrow[\text{15 min}]{\text{HCl-CH}_3\text{ONO}}$ 25 ·C $\xrightarrow[\text{8 hr}]{\text{HCl-CH}_3\text{ONO}}$ 25·C $\xrightarrow[\text{15min}]{\text{N}_2}$ Normal temp. | 410 | 1.0 |
| Example 17 | PdCl$_2$-CuCl$_2$-(NH$_4$)$_6$Mo$_7$O$_{24}$/C | Same as above | Normal temp. $\xrightarrow[\text{15min}]{\text{HCl}}$ 60 ·C $\xrightarrow[\text{8 hr}]{\text{HCl}}$ 60 ·C $\xrightarrow[\text{15min}]{\text{N}_2}$ Normal temp. | 440 | 3.0 |
| Example 18 | PdCl$_2$-CuCl$_2$-(NH$_4$)$_6$Mo$_7$O$_{24}$/C | Same as above | Normal temp. $\xrightarrow[\text{2 hr}]{\text{HCl}}$ 250 ·C $\xrightarrow[\text{8 hr}]{\text{HCl}}$ 250 ·C $\xrightarrow[\text{1 hr}]{\text{N}_2}$ Normal temp. | 469 | 2.4 |

Table 1 (contd)

| No. | Catalyst | Regeneration of catalyst | | DMC formed rate Y (g/l·hr) | Activity decreased coefficient Da (hr$^{-1}$) |
|---|---|---|---|---|---|
| | | H$_2$ treatment | HCl treatment | | |
| Comparative example 1 | PdCl$_2$-CuCl$_2$-(NH$_4$)$_6$Mo$_7$O$_{24}$/C | ——— | Normal temp. $\xrightarrow[\text{0.5hr}]{\text{HCl}}$ 120 ·C $\xrightarrow[\text{8 hr}]{\text{HCl}}$ 120 ·C $\xrightarrow[\text{0.5hr}]{\text{N}_2}$ Normal temp. | 198 | 5.4 |
| Comparative example 2 | PdCl$_2$-CuCl$_2$-(NH$_4$)$_6$Mo$_7$O$_{24}$/C | Normal temp. $\xrightarrow[\text{2 hr}]{\text{H}_2}$ 400 ·C $\xrightarrow[\text{2 hr}]{\text{H}_2}$ 400 ·C $\xrightarrow[\text{2 hr}]{\text{H}_2}$ Normal temp. | ——— | 56 | 4.1 |
| Com.ex 3 | PdCl$_2$/C | ——— | ——— | 6 | — |
| Com.ex 4 | PdCl$_2$-CuCl$_2$/C | ——— | ——— | 5 | — |
| Com.ex 5 | PdCl$_2$-CuCl$_2$-(NH$_4$)$_6$Mo$_7$O$_{24}$/C | ——— | ——— | 10 | — |
| Com.ex 6 | PdCl$_2$-CuCl$_2$-(NH$_4$)$_6$Mo$_7$O$_{24}$-KCl/C | ——— | ——— | 7 | — |

**Claims**

1. A process for preparing diester of carbonic acid from carbon monoxide and a nitrite in the presence of a solid catalyst in which at least one selected from the group consisting of a platinum group metal and a platinum group metal compound is carried on a carrier, characterized in that a deactivated catalyst is regenerated by simultaneously or successively treating with hydrogen and with hydrogen chloride, and used repeatedly.

2. The process according to Claim 1, wherein other metal compound of a metal selected from the group consisting of iron, copper, bismuth, cobalt, nickel, tin, vanadium, molybdenum, tungsten, an alkali metal and an alkaline earth metal is further carried on the solid catalyst.

3. The process according to Claim 1, wherein treatment with hydrogen is carried out by flowing a gas containing hydrogen with a space velocity of 50 to 20000 hr$^{-1}$ under a pressure of 1 to 100 atm at a temperature of 70 to 500 °C for several hours to several ten hours.

4. The process according to Claim 3, wherein the gas contains hydrogen at a concentration of 5 to 100 %.

5. The process according to Claim 1, wherein treatment with hydrogen is carried out by flowing a gas containing hydrogen with a space velocity of 100 to 10000 hr$^{-1}$ under a pressure of 1 to 20 atm at a temperature of 100 to 450 °C.

6. The process according to Claim 5, wherein the gas contains hydrogen at a concentration of 20 to 100 %.

7. The process according to Claim 1, wherein treatment with hydrogen chloride is carried out by flowing a gas containing hydrogen chloride with a space velocity of 40 to 20000 hr$^{-1}$ under a pressure of 1 to 20 atm at a temperature of 15 to 500 °C for 0.01 to 100 hours.

8. The process according to Claim 7, wherein the gas contains hydrogen chloride at a concentration of 20 ppm to 100 %.

9. The process according to Claim 1, wherein treatment with hydrogen chloride is carried out by flowing a gas containing hydrogen chloride with a space velocity of 100 to 10000 hr$^{-1}$ under a pressure of 1 to 5 atm at a temperature of 50 to 400 °C for 0.1 to 50 hours.

10. The process according to Claim 7, wherein the gas contains hydrogen chloride at a concentration of 100 to 50000 ppm.

11. The process according to Claim 1, wherein treatment with hydrogen chloride is carried out by flowing a mixed gas containing hydrogen chloride and a nitrite.

12. The process according to Claim 11, wherein the treatment with the mixed gas is carried out with the nitrite concentration of 0.5 to 20 %, a space velocity of the mixed gas of 10 to 20000 hr$^{-1}$ under the pressure of 1 to 20 atm at the temperature of 15 to 300 °C, more preferably 25 to 250 °C for 0.01 to 100 hours.

13. The process according to Claim 1, wherein regeneration of the catalyst is carried out by flowing a gas containing hydrogen at a hydrogen concentration of 5 to 100 % with a space velocity of 50 to 20000 hr$^{-1}$ under a pressure of 1 to 100 atm at a temperature of 70 to 500 °C for several hours to several ten hours and then flowing a gas containing hydrogen chloride at a hydrogen chloride concentration of 20 ppm to 100 % with a space velocity of 40 to 20000 hr$^{-1}$ under a pressure of 1 to 20 atm at a temperature of 15 to 500 °C for 0.01 to 100 hours.

14. The process according to Claim 1, wherein regeneration of the catalyst is carried out by flowing a gas containing hydrogen at a hydrogen concentration of 20 to 100 % with a space velocity of 100 to 10000 hr$^{-1}$ under a pressure of 1 to 20 atm at a temperature of 100 to 450 °C for several hours to several ten hours and then flowing a gas containing hydrogen chloride at a hydrogen chloride concentration of 100 to 50000 ppm with a space velocity of 100 to 10000 hr$^{-1}$ under a pressure of 1 to 5 atm at a temperature of 50 to 400 °C for 0.1 to 50 hours.

**Patentansprüche**

1. Verfahren zur Herstellung eines Diesters von Kohlensäure aus Kohlenmonoxid und einem Nitrit in der Gegenwart eines festen Katalysators, bei dem zumindest ein Element, ausgewählt aus der Gruppe, bestehend aus einem Metall der Platin-Gruppe und einer Metall-Verbindung der Platin-Gruppe auf einem Träger getragen wird, dadurch **gekennzeichnet,** daß ein deaktivierter Katalysator durch gleichzeitiges oder aufeinanderfolgendes Behandeln mit Wasserstoff und Chlorwasserstoff regeneriert und wiederholt verwendet wird.

2. Verfahren nach Anspruch 1, worin eine andere Metall-Verbindung aus einem Metall, ausgewählt aus der Gruppe, bestehend aus Eisen, Kupfer, Wismut, Kobalt, Nickel, Zinn, Vanadium, Molybdän, Wolfram, einem Alkalimetall und einem Erdalkalimetall, weiterhin auf dem festen Katalysator getragen wird.

3. Verfahren nach Anspruch 1, worin die Behandlung mit Wasserstoff durch Fließen eines wasserstoffhaltigen Gases mit einer Raumgeschwindigkeit von 50 bis 20 000 h⁻¹ unter einem Druck von 1 bis 100 atm bei einer Temperatur von 70 bis 500°C für mehrere Stunden bis mehrere 10 h durchgeführt wird.

4. Verfahren nach Anspruch 3, worin das Gas Wasserstoff mit einer Konzentration von 5 bis 100 % enthält.

5. Verfahren nach Anspruch 1, worin die Behandlung mit Wasserstoff durch Fließen eines Wasserstoff-haltigen Gases mit einer Raumgeschwindigkeit von 100 bis 10 000 h⁻¹ unter einem Druck von 1 bis 20 atm bei einer Temperatur von 100 bis 450°C durchgeführt wird.

6. Verfahren nach Anspruch 5, worin das Gas Wasserstoff in einer Konzentration von 20 bis 100 % enthält.

7. Verfahren nach Anspruch 1, worin die Behandlung mit Chlorwasserstoff durch Fließen eines Chlorwasserstoff-haltigen Gases mit einer Raumgeschwindigkeit von 50 bis 20 000 h⁻¹ unter einem Druck von 1 bis 20 atm bei einer Temperatur von 15 bis 500°C für 0,01 bis 100 h durchgeführt wird.

8. Verfahren nach Anspruch 7, worin das Gas Chlorwasserstoff bei einer Konzentration von 20 ppm bis 100 % enthält.

9. Verfahren nach Anspruch 1, worin die Behandlung mit Chlorwasserstoff durch Fließen eines Chlorwasserstoff-haltigen Gases mit einer Raumgeschwindigkeit von 100 bis 10 000 h⁻¹ unter einem Druck von 1 bis 5 atm bei einer Temperatur von 50 bis 400°C für 0,1 bis 50 h durchgeführt wird.

10. Verfahren nach Anspruch 7, worin das Gas Chlorwasserstoff in einer Konzentration von 100 bis 50 000 ppm enthält.

11. Verfahren nach Anspruch 1, worin die Behandlung mit Chlorwasserstoff durch Fließen eines Mischgases, umfassend Chlorwasserstoff und ein Nitrit, durchgeführt wird.

12. Verfahren nach Anspruch 11, worin die Behandlung mit dem Mischgas mit einer Nitrit-Konzentration von 0,5 bis 20 %, einer Raumgeschwindigkeit des Mischgases von 10 bis 20 000 h⁻¹ unter dem Druck von 1 bis 20 atm bei der Temperatur von 15 bis 300°C, mehr bevorzugt 25 bis 250°C für 0,01 bis 100 h durchgeführt wird.

13. Verfahren nach Anspruch 1, worin die Regenerierung des Katalysators durch Fließen eines Wasserstoff-haltigen Gases mit einer Wasserstoff-Konzentration von 5 bis 100 % mit einer Raumgeschwindigkeit von 50 bis 20 000 h⁻¹ unter einem Druck von 1 bis 100 atm bei einer Temperatur von 70 bis 500°C für mehrere Stunden bis mehrere 10 h und durch anschließendes Fließen eines Chlorwasserstoff-haltigen Gases bei einer Chlorwasserstoff-Konzentration von 20 ppm bis 100 % mit einer Raumgeschwindigkeit von 40 bis 20 000 h⁻¹ unter einem Druck von 1 bis 20 atm bei einer Temperatur von 15 bis 500°C für 0,01 bis 100 h durchgeführt wird.

14. Verfahren nach Anspruch, worin die Regeneration des Katalysators durch Fließen eines Wasserstoff-haltigen Gases bei einer Wasserstoff-Konzentration von 20 bis 100 % mit einer Raumgeschwindigkeit von 100 bis 10 000 h⁻¹ unter einem Druck von 1 bis 20 atm bei einer Temperatur von 100 bis 450°C für mehrere Stunden bis mehrere zehn Stunden und durch anschließendes Fließen eines Chlorwasserstoff-haltigen Gases mit einer Chlorwasserstoff-Konzentration von 100 bis 50 000 ppm mit einer Raumgeschwindigkeit von 100 bis 10 000 h⁻¹ unter einem Druck von 1 bis 5 atm bei einer Temperatur von 50 bis 400°C für 0,1 bis 50 h durchgeführt wird.

**EP 0 565 076 B1**

**Revendications**

1.  Procédé de préparation d'un diester d'acide carbonique à partir de monoxyde de carbone et d'un nitrite en présence d'un catalyseur solide dans lequel au moins un membre choisi parmi le groupe se composant d'un métal du groupe du platine et un composé d'un métal du groupe du platine est porté par un support, caractérisé en ce qu'un catalyseur désactivé est régénéré par traitement simultané ou successif à l'aide d'hydrogène et à l'aide d'acide chlorhydrique, et est utilisé de manière répétée.

2.  Procédé selon la revendication 1, caractérisé en ce qu'en outre un autre composé de métal d'un métal choisi parmi le groupe se composant du fer, du cuivre, du bismuth, du cobalt, du nickel, de l'étain, du vanadium, du molybdène, du tungstène, d'un métal alcalin et d'un métal alcalino-terreux est porté sur le catalyseur solide.

3.  Procédé selon la revendication 1, caractérisé en ce que le traitement à l'aide d'hydrogène est effectué en faisant s'écouler un gaz contenant de l'hydrogène à une vitesse spatiale de 50 à 20 000 hr$^{-1}$, sous une pression de 1 à 100 atmosphères à une température de 70 à 500°C pendant une durée allant de plusieurs heures à plusieurs dizaines d'heures.

4.  Procédé selon la revendication 3, caractérisé en ce que le gaz contient de l'hydrogène à une concentration de 5 à 100 %.

5.  Procédé selon la revendication 1, caractérisé en ce que le traitement à l'aide d'hydrogène est effectué en faisant s'écouler un gaz contenant de l'hydrogène à une vitesse spatiale de 100 à 10 000 hr$^{-1}$ sous une pression de 1 à 20 atmosphères à une température de 100 à 450°C.

6.  Procédé selon la revendication 5, caractérisé en ce que le gaz contient de l'hydrogène à une concentration de 20 à 100 %.

7.  Procédé selon la revendication 1, caractérisé en ce que le traitement à l'aide d'acide chlorhydrique est effectué en faisant s'écouler un gaz contenant de l'acide chlorhydrique à une vitesse spatiale de 40 à 20 000 hr$^{-1}$, sous une pression de 1 à 20 atmosphères à une température de 15 à 500°C pendant une durée allant de 0,01 à 100 heures.

8.  Procédé selon la revendication 7, caractérisé en ce que le gaz contient de l'acide chlorhydrique à une concentration de 20 ppm à 100 %.

9.  Procédé selon la revendication 1, caractérisé en ce que le traitement à l'aide d'acide chlorhydrique est effectué en faisant s'écouler un gaz contenant de l'acide chlorhydrique à une vitesse spatiale de 100 à 10 000 hr$^{-1}$, sous une pression de 1 à 5 atmosphères à une température de 50 à 400°C pendant une durée allant de 0,1 à 50 heures.

10. Procédé selon la revendication 7, caractérisé en ce que le gaz contient de l'acide chlorhydrique à une concentration de 100 à 50 000 ppm.

11. Procédé selon la revendication 1, caractérisé en ce que le traitement à l'aide d'acide chlorhydrique est effectué en faisant s'écouler un gaz mixte contenant de l'acide chlorhydrique et un nitrite.

12. Procédé selon la revendication 11, caractérisé en ce que le traitement à l'aide du gaz mixte est effectué à la concentration de nitrite de 0,5 à 20 %, une vitesse spatiale du gaz mixte de 10 à 20 000 hr$^{-1}$ sous la pression de 1 à 20 atmosphères à la température de 15 à 300°C, plus préférablement de 25 à 250°C, pendant une durée allant de 0,01 à 100 heures.

13. Procédé selon la revendication 1, caractérisé en ce que la régénération du catalyseur est effectuée en faisant s'écouler un gaz contenant de l'hydrogène à une concentration d'hydrogène de 5 à 100 % à une vitesse spatiale de 50 à 20 000 hr$^{-1}$, sous une pression de 1 à 100 atmosphères à une température de 70 à 500°C pendant une durée allant de plusieurs heures à plusieurs dizaines d'heures, et ensuite en faisant s'écouler un gaz contenant de l'acide chlorhydrique à une concentration d'acide chlorhydrique de 20 ppm à 100 %, à une vitesse spatiale de 40 à 20 000 hr$^{-1}$, sous une pression de 1 à 20 atmosphères à une température de 15 à 500°C pendant une durée allant de 0,01 à 100 heures.

14. Procédé selon la revendication 1, caractérisé en ce que la régénération du catalyseur est effectuée en faisant s'écouler un gaz contenant de l'hydrogène à une concentration d'hydrogène de 20 à 100 % à une vitesse spatiale de 100 à 10 000 hr$^{-1}$, sous une pression de 1 à 20 atmosphères à une température de 100 à 450°C pendant une durée allant de plusieurs heures à plusieurs dizaines d'heures, et ensuite en faisant s'écouler un gaz contenant de l'acide chlorhydrique à une concentration d'acide chlorhydrique de 100 à 50 000 ppm à une vitesse spatiale de 100 à 10 000 hr$^{-1}$, sous une pression de 1 à 5 atmosphères à une température de 50 à 400°C pendant une durée allant de 0,1 à 50 heures.